# FASCICULE DE BREVET EUROPEEN

(11) **EP 2 459 100 B1**
(45) Date de publication et mention de la délivrance du brevet: **12.03.2014**
(21) Numéro de dépôt: 10745428.2
(22) Date de dépôt: 30.07.2010
(51) Int. Cl.: A61B 17/29, A61B 19/00

(54) **MANIPULATEUR ERGONOMIQUE ET SEMI-AUTOMATIQUE ET APPLICATIONS AUX INSTRUMENTS POUR CHIRURGIE MINI-INVASIVE**
ERGONOMISCHER UND HALBAUTOMATISCHER MANIPULATOR SOWIE ANWENDUNGEN FÜR INSTRUMENTE ZUR MINIMAL-INVASIVEN CHIRURGIE
ERGONOMIC AND SEMI-AUTOMATIC MANIPULATOR, AND APPLICATIONS TO INSTRUMENTS FOR MINIMALLY INVASIVE SURGERY

(30) Priorité: 31.07.2009 FR 0955382
(43) Date de publication de la demande: 06.06.2012
(73) Titulaire: Dexterite Surgical, 74000 Annecy (FR)
(72) Inventeur: BARRIER, Pascal, F-74000 Annecy (FR); OLLAGNIER, Jérémy, F-74960 Meythet (FR); ROSSET-LANCHET, Rémi, F-74000 Annecy (FR); MELENNEC épouse BARTHOD, Christine, F-74370 Naves Parmelan (FR); GIORDANO, Max, F-74940 Annecy Le Vieux (FR)
(74) Mandataire: Poncet, Jean-François
(86) Numéro de dépôt international: PCT/IB2010/053477
(87) Numéro de publication internationale: WO 2011/013103

(56) Documents cités:
- JP-A- 2001 276 091
- US-A1- 2002 040 217
- US-A1- 2004 260 334
- US-A1- 2005 222 587
- US-A1- 2008 039 255

## Description

### DOMAINE TECHNIQUE DE L'INVENTION

La présente invention concerne les dispositifs de guidage et de manipulation permettant de commander, depuis l'extérieur d'une zone d'intervention, les mouvements d'un instrument de manipulation situé à l'intérieur de la zone d'intervention.

En particulier, ces dispositifs de guidage et de manipulation permettent de commander un instrument chirurgical dans les applications de chirurgie mini-invasive pratiquées sous endoscopie.

Dans ces applications, il est nécessaire de pouvoir déplacer et commander un instrument chirurgical pour effectuer diverses opérations telles qu'une suture, le serrage d'un noeud, une fine dissection de tissus. Il s'agit alors d'opérations délicates, précises, dans lesquelles les mouvements à réaliser sont complexes.

On a déjà proposé des dispositifs de guidage et de manipulation par robot télécommandé, par exemple le dispositif décrit dans le document US 5,797,900. Dans ce cas, un bras maître, actionnable par un opérateur, est mécaniquement complètement séparé et distant d'un bras esclave qui porte l'instrument chirurgical. Le bras esclave est porté par un support au voisinage de la table d'opération. Le bras maître est situé à un poste de commande distant, muni de moyens de visualisation, les deux postes étant reliés par des lignes de transmission de signaux de visualisation et de commande. Un tel dispositif est particulièrement complexe, onéreux et encombrant, comportant un poste de manipulation distant pour télécommander un poste esclave portant l'instrument chirurgical.

L'invention concerne plus spécialement les dispositifs portatifs de guidage et de manipulation, dans lesquels l'instrument chirurgical est placé en bout d'un manipulateur portable ayant essentiellement un bras de liaison à extrémité proximale et extrémité distale. On connaît des manipulateurs portatifs dans lesquels l'extrémité proximale du bras de liaison porte une unité de commande ayant une poignée apte à être tenue par une main. Des organes de commande, montés sur la poignée, sont aptes à être actionnés par au moins un doigt de la main tenant la poignée. Une unité de travail est montée sur l'extrémité distale du bras de liaison, et comporte un support d'outils apte à supporter un outil chirurgical. Le support d'outils est monté inclinable dans l'unité de travail, par rapport au bras de liaison, par rotation autour d'un axe transversal d'inclinaison. Les organes de commande permettent ainsi de commander l'orientation du support d'outils selon une direction d'inclinaison par rotation autour de l'axe transversal d'inclinaison. Les organes de commande permettent aussi de commander l'orientation de la direction d'inclinaison autour de l'axe longitudinal du bras de liaison, afin de communiquer à l'outil une orientation pouvant être choisie dans un cône autour de l'axe longitudinal du bras de liaison. Les organes de commande permettent en outre de commander la rotation propre du support d'outils autour de la direction d'inclinaison.

Un tel dispositif portatif est par exemple décrit dans le document US 2005/0222587 A1, et comprend :
- une unité de commande, ayant une poignée apte à être tenue par une main,
- des organes de commande, montés sur la poignée, et aptes à être actionnés par au moins un doigt de la main tenant la poignée,
- un bras de liaison, s'étendant le long d'un axe longitudinal, ayant une extrémité proximale dans laquelle est montée l'unité de commande, et ayant une extrémité distale,
- une unité de travail, montée sur l'extrémité distale du bras de liaison, et comportant un support d'outils apte à supporter un outil,
- le support d'outils étant monté inclinable dans l'unité de travail, par rapport au bras de liaison, par rotation autour d'un axe transversal d'inclinaison,
- les organes de commande permettant de commander l'inclinaison transversale du support d'outils selon une direction d'inclinaison par rotation autour de l'axe transversal d'inclinaison,
- les organes de commande permettant de commander la rotation propre du support d'outils autour de la direction d'inclinaison ;
en outre :
- dans sa rotation d'inclinaison transversale, le support d'outils est entraîné par au moins un premier actionneur d'inclinaison piloté par un premier des organes de commande de la poignée,
- dans le mouvement de rotation propre du support d'outils autour de la direction d'inclinaison, le manipulateur est piloté par un second des organes de commande de la poignée, qui commande un ou plusieurs actionneurs,
- dans le mouvement d'orientation de la direction d'inclinaison autour de l'axe longitudinal du bras de liaison, le manipulateur est piloté pour réaliser une rotation autour de l'axe longitudinal par la sollicitation d'un organe d'entraînement motorisé en rotation de la poignée par rapport au reste du manipulateur autour de l'axe longitudinal du bras de liaison.

Grâce à l'utilisation d'actionneurs pilotés par des organes de commande disposés sur la poignée, la main tenant la poignée peut porter le manipulateur et le tenir en une position bien définie et aisément contrôlable par l'opérateur, qui contrôle naturellement ainsi la position de l'outil chirurgical. Simultanément, les mouvements de rotation propre et d'inclinaison du support d'outils en bout du manipulateur sont commandés par l'actionnement des organes de commande montés sur la poignée, et ces organes de commande peuvent être sollicités par des mouvements de force et d'amplitude faibles des doigts de la main tenant la poignée, assurant une bonne dissociation entre le maintien du manipulateur pour fixer la position de l'outil chirurgical et la commande des mouvements du support d'outils autour de sa position définie par la position du manipulateur.

Simultanément, les différents mouvements possibles d'orientation de la direction d'inclinaison autour de l'axe longitudinal du bras de liaison permettent de donner à l'outil toutes positions nécessaires dans un cône prolongeant le bras de liaison.

### EXPOSE DE L'INVENTION

Il s'avère toutefois qu'avec un tel dispositif, la transmission des différents mouvements du porte-outil est encombrante, notamment par la disposition en cascade de plusieurs articulations le long de l'axe longitudinal, et par le fait qu'aucun moyen n'est indiqué pour réaliser l'ensemble des transmissions de mouvement dans un faible diamètre de partie distale de manipulateur. Un besoin existe donc de concevoir d'autres moyens de transmission assurant simultanément un encombrement réduit et une rigidité suffisante des transmissions qui le nécessitent.

Pour cela, la présente invention prévoit que les mouvements de rotation propre et d'ouverture-fermeture éventuelle de pince sont transmis au support d'outils par des engrenages et tubes ou arbres logés dans l'unité de travail, tandis que les mouvements d'inclinaison autour de l'axe transversal sont transmis par des câbles.

Un intérêt des engrenages et tubes ou arbres est d'assurer une transmission d'efforts rigide, pour les mouvements qui nécessitent cette propriété, tandis que la transmission par câbles pour le mouvement d'inclinaison permet de réduire simultanément l'encombrement du dispositif.

En pratique, on peut avantageusement prévoir que le mouvement de rotation propre du support d'outils est assuré par un tube d'entrée, entraîné lui-même en rotation par un actionneur de rotation propre, et portant un pignon conique d'extrémité qui lui-même entraîne en rotation un pignon conique latéral rotatif autour d'un axe transversal et qui entraîne un pignon conique axial solidaire du support d'outils.

De même, on peut en outre prévoir que l'outil est une pince, et que :
- un actionneur de pince est couplé à un arbre d'entrée axial orienté le long de l'axe longitudinal du bras de liaison,
- l'arbre d'entrée axial porte un pignon conique d'extrémité qui entraîne un pignon conique latéral rotatif autour d'un axe transversal et entraînant un pignon conique monté en bout d'un arbre de sortie fileté et calé axialement sur lequel est monté un écrou solidaire d'un mors mobile de l'outil.

Cette disposition permet d'assurer une grande force de serrage, utile en particulier lorsque la pince est un porte-aiguille.

Dans le cas d'une pince, il est en outre avantageux de prévoir que la pince comporte un mors fixe et un mors mobile : le mors fixe facilite le positionnement d'une aiguille avant de fermer le mors mobile.

Dans le cas d'un outil en forme de pince, on peut en outre prévoir que le support d'outils comprend un arceau monté pivotant autour d'un axe transversal sur l'extrémité distale du bras de liaison et solidaire d'une poulie sollicitée en rotation par un câble lui-même sollicité par un actionneur d'inclinaison.

Pour améliorer encore la dissociation entre la commande du mouvement de rotation propre du support d'outils et tous les autres mouvements du support d'outils, et notamment le maintien du manipulateur en position, on peut avantageusement prévoir que le second des organes de commande comprend un premier organe d'entrée dont l'actionnement provoque la rotation propre du support d'outils dans un premier sens de rotation, et un second organe d'entrée dont l'actionnement provoque la rotation propre du support d'outils dans le second sens de rotation. On donne ainsi à l'opérateur une grande facilité d'inclinaison du support d'outils en bout du bras de liaison, par des mouvements naturels des doigts de la main.

Selon un mode de réalisation avantageux, le premier des organes de commande peut comprendre un troisième organe d'entrée dont la sollicitation provoque une augmentation positive de l'angle d'inclinaison, et un quatrième organe d'entrée dont la sollicitation provoque une augmentation négative de l'angle d'inclinaison.

Selon une première réalisation, les organes d'entrée des premier et second organes de commande peuvent être de type « tout ou rien », et peuvent piloter chacun le mouvement du support d'outils dans un sens respectif selon une vitesse de rotation sensiblement constante.

En alternative, on peut prévoir que les organes d'entrée sont de type progressif, pilotant chacun le mouvement du support d'outils selon une vitesse de rotation variable entre une vitesse rapide et une vitesse lente. On réalise ainsi un bon compromis entre la vitesse d'actionnement et la précision.

De préférence, on pourra prévoir que les organes d'entrée sont de type « tout ou rien » à mode pas à pas se mettant en mode continu à vitesse plus rapide par maintien de sollicitation.

Plus avantageusement, on peut prévoir que le premier des organes de commande comprend en outre une possibilité de sollicitation de remise à zéro, qui ramène le support d'outils dans l'axe du bras support. De la sorte, on donne à l'opérateur une grande facilité de repérage de la position, à partir de la position neutre en bout du bras de liaison.

Dans le cas d'un outil de type pince, le manipulateur peut avantageusement comprendre en outre, sur la poignée, un organe de commande de pince dont l'actionnement pilote au moins un actionneur de pince qui produit sélectivement l'ouverture et la fermeture de la pince. Ainsi, l'actionnement de la pince est également réalisé par un mouvement aisé d'un doigt de la main, sans interférence avec le maintien du manipulateur et avec les autres mouvements du support d'outils.

Dans ce cas, l'organe de commande de pince peut comprendre notamment une première position de pince ouverte, au moins une seconde position de pince fermée à faible serrage, et au moins une troisième position de pince fermée à fort serrage.

En complément ou en alternative, on peut prévoir en outre un actionneur de rotation de bras réalisant la rotation propre du bras de liaison autour de son axe longitudinal par rapport à la poignée, ledit actionneur de rotation propre de bras étant piloté par le troisième organe de commande de la poignée.

Selon une autre réalisation avantageuse, le manipulateur peut comprendre, entre la poignée et le bras de liaison, un bras de positionnement par lequel l'opérateur peut modifier et fixer la position angulaire et/ou spatiale relative à la poignée par rapport au bras de liaison.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est un schéma de principe illustrant la structure générale d'un manipulateur selon un mode de réalisation de la présente invention, en position d'utilisation ;
- la figure 2 illustre le manipulateur de la figure 1, avec la poignée légèrement pivotée ;
- la figure 3 illustre le manipulateur de la figure 1, en position pivotée autour du trocart ;
- la figure 4 est une vue schématique du manipulateur de la figure 3, avec la poignée pivotée ;
- la figure 5 est une vue générale du manipulateur des figures précédentes, illustrant le mouvement d'inclinaison du support d'outils ;
- la figure 6 illustre le mouvement de rotation propre du support d'outils ;
- la figure 7 illustre un mode de réalisation du manipulateur des figures précédentes, et montre les différents mouvements de l'outil ;
- la figure 8 illustre un autre mode de réalisation pour obtenir les mouvements de l'outil ;
- la figure 9 illustre un autre mode de réalisation pour obtenir les mouvements de l'outil ;
- la figure 10 illustre un autre mode de réalisation pour obtenir les mouvements de l'outil ;
- la figure 11 illustre un mode de réalisation avantageux pour obtenir les mouvements de l'outil ;
- la figure 12 illustre un autre mode de réalisation du manipulateur, avec un moyen élastique de rappel axial entre le trocart et le corps de manipulateur ;
- la figure 13 est une vue de côté en coupe illustrant une unité de travail selon un mode de réalisation de la présente invention ;

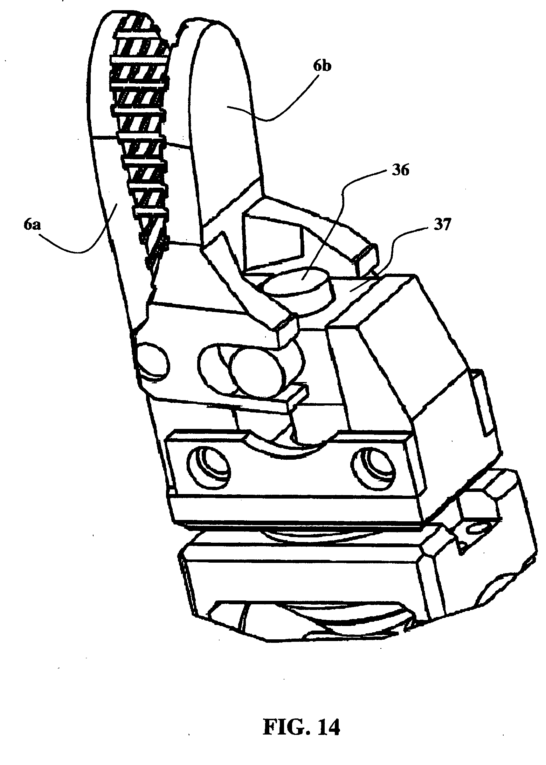

- la figure 14 est une vue en perspective illustrant partiellement l'unité de travail selon la figure 13 ;
- la figure 15 illustre un mode de réalisation des moyens d'entraînement en inclinaison du support d'outils ;
- la figure 16 est une vue en perspective générale d'un manipulateur selon un mode de réalisation de l'invention ;
- la figure 17 est une vue en perspective du manipulateur de la figure 16, capot d'unité de commande enlevé ;
- la figure 18 illustre, en vue de côté en coupe, les moyens de transmission des mouvements selon un autre mode de réalisation d'une unité de travail de l'invention ;
- la figure 19 est une vue schématique générale en perspective d'un manipulateur selon le mode de réalisation de l'invention ;
- la figure 20 illustre les étapes d'un mouvement de rotation manuelle du corps de manipulateur à l'aide d'une poignée articulée ;
- la figure 21 illustre, en perspective, les différentes étapes d'un mouvement de rotation propre axial motorisé d'un corps de manipulateur par rapport à la poignée ;
- la figure 22 illustre deux positions angulaires du manipulateur par rapport à la poignée dans un mode de réalisation particulier à moyens manuels d'orientation de la direction d'inclinaison ;
- la figure 23 est une vue partielle de côté en coupe des moyens d'orientation manuels de la figure 22 ;
- la figure 24 est une vue en perspective des moyens d'orientation manuels selon une variante de l'invention ;
- la figure 25 illustre schématiquement un manipulateur selon un autre mode de réalisation de l'invention, à liaison déformable entre la poignée et le corps de manipulateur ;
- la figure 26 illustre un autre mode de réalisation du manipulateur selon l'invention, à liaison articulée entre la poignée et le corps de manipulateur ; et
- la figure 27 illustre un exemple de positionnement d'organes d'entrée sur une poignée.

### DESCRIPTION DES MODES DE REALISATION PREFERES

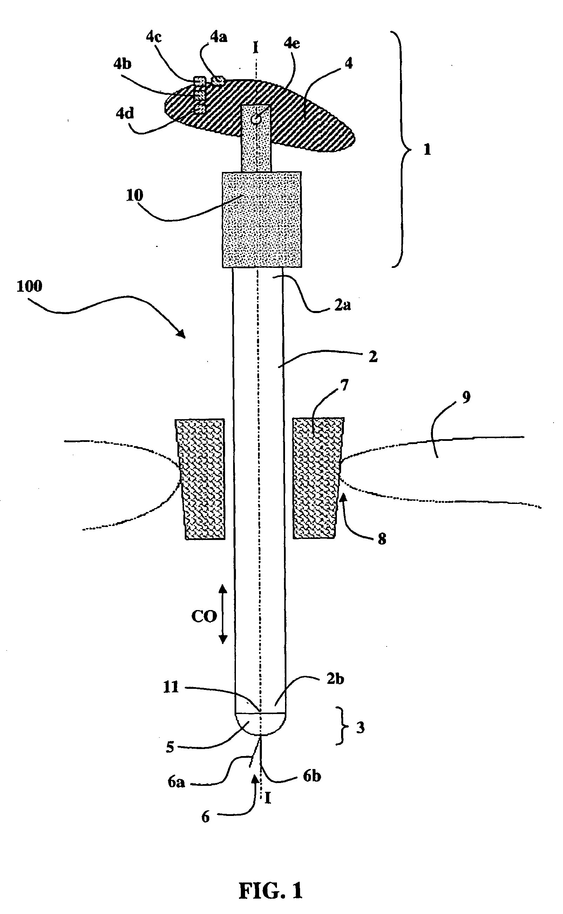

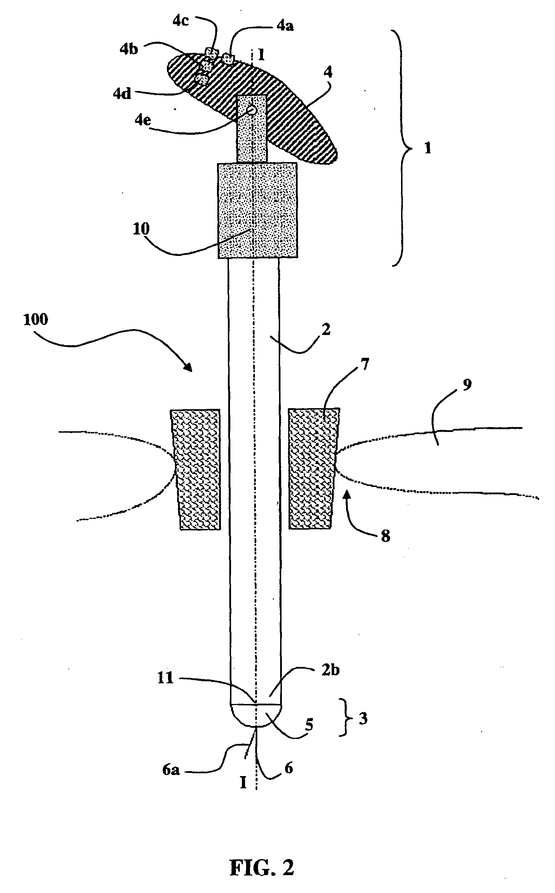

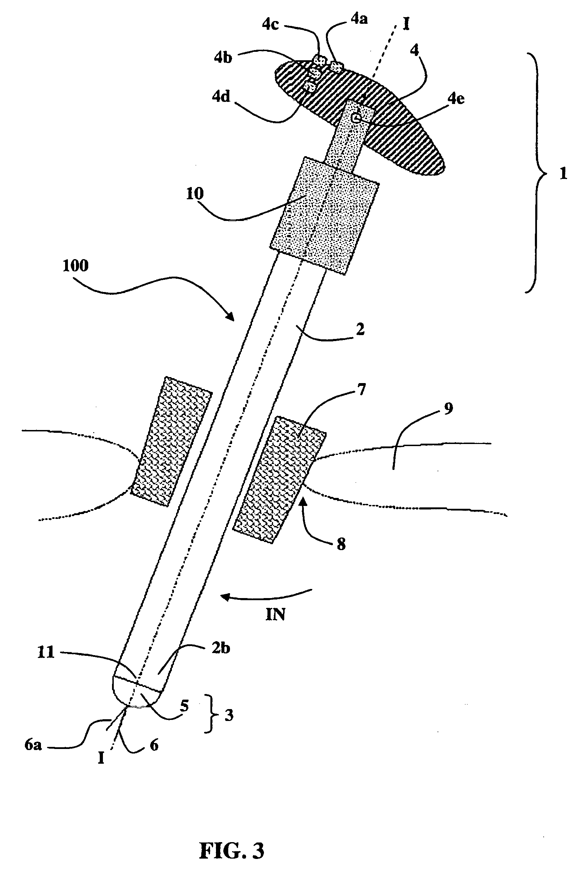

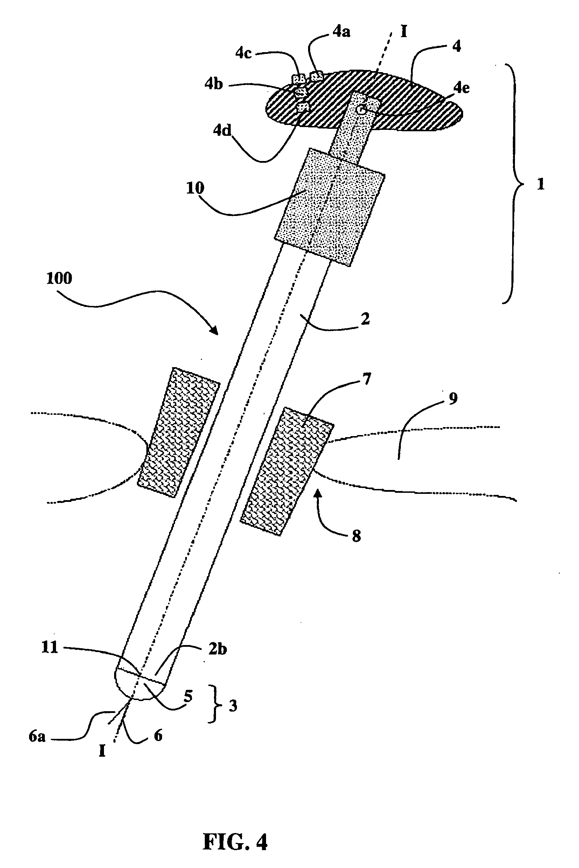

On considère tout d'abord la structure générale d'un manipulateur selon l'invention, tel qu'illustré sur les figures 1 à 12.

Un tel manipulateur 100 comprend de façon générale une unité de commande 1, un bras de liaison 2, et une unité de travail 3.

L'unité de commande 1 est montée sur l'extrémité proximale 2a du bras de liaison 2, tandis que l'unité de travail 3 est montée sur l'extrémité distale 2b du bras de liaison 2.

L'unité de commande 1 comprend une poignée 4 apte à être tenue par une main d'un opérateur, et un corps de commande 10 contenant divers moyens d'entraînement pour produire les mouvements appropriés dans l'unité de travail 3.

L'unité de travail 3 comporte un support d'outils 5 apte à supporter un outil 6. Sur les figures, on a illustré un outil 6 en forme de pince à deux branches 6a et 6b.

La poignée 4 comporte des organes de commande, par exemple un premier organe de commande 4a, un second organe de commande 4b, un troisième organe de commande 4c, et un organe de commande de pince 4d.

Comme dans les dispositifs connus, le bras de liaison 2 passe dans un trocart 7 assurant la liaison et le passage dans une ouverture 8 pratiquée dans la paroi corporelle 9. L'unité de commande 1 reste à l'extérieur du corps du patient, tandis que l'unité de travail 3 pénètre à l'intérieur du corps du patient pour atteindre la zone d'intervention où l'outil 6 doit exécuter des mouvements pilotés depuis l'unité de commande 1.

Sur les figures 1 à 4, l'outil 6 et le support d'outils 5 sont en alignement avec l'axe longitudinal I-I du bras de liaison 2. Comme on le voit sur ces figures, l'opérateur peut positionner l'unité de travail de façon précise dans la zone opératoire par coulissement CO du bras de liaison 2 dans le trocart 7, et par inclinaison IN du bras de liaison 2 et du trocart 7 dans l'ouverture 8 de la paroi corporelle 9.

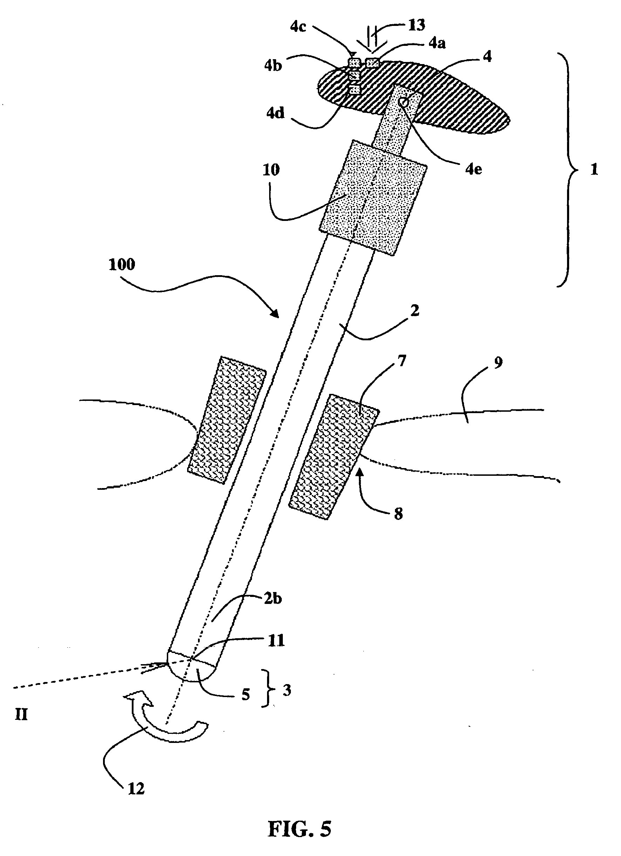

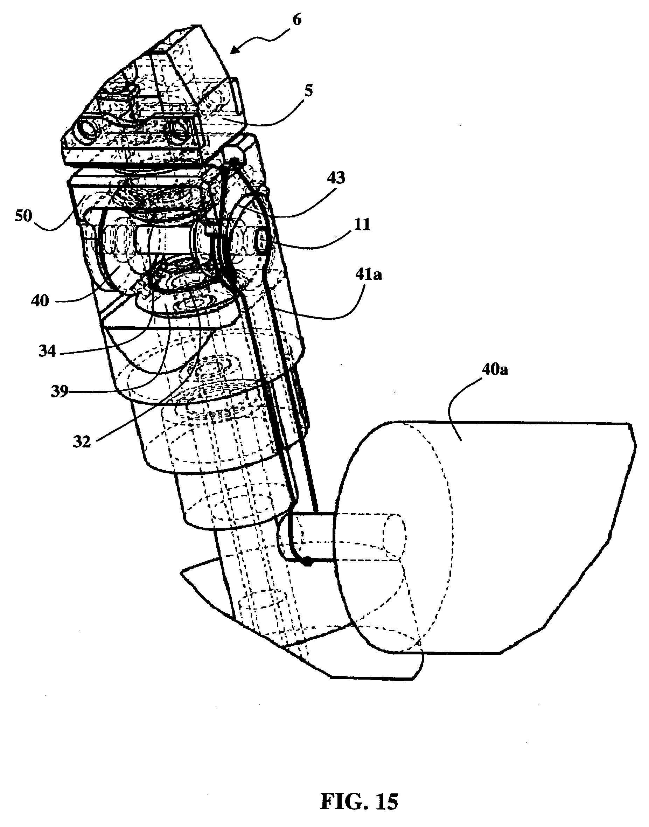

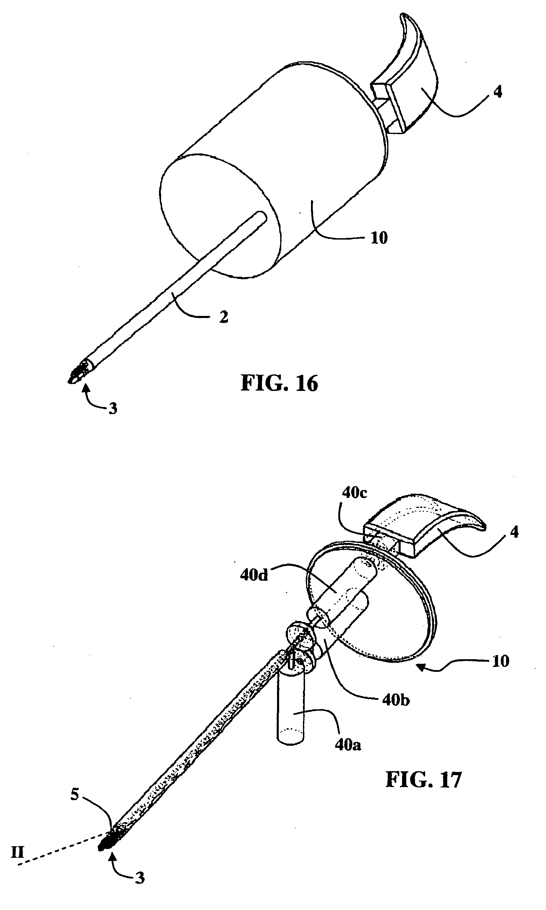

Sur la figure 5, on a illustré un premier mouvement du support d'outils 5 à savoir un mouvement d'inclinaison 12 autour d'un axe transversal d'inclinaison 11. Ce mouvement est obtenu par la sollicitation 13 du premier organe de commande 4a de la poignée 4, qui commande un actionneur d'inclinaison logé dans le corps de commande 10. A titre d'illustration, on pourra se reporter à la figure 17, illustrant l'actionneur d'inclinaison 40a, relié à l'unité de travail 3 par un câble de transmission d'inclinaison 41a illustré sur la figure 15.

Sur la figure 5, par actionnement 13 du premier organe de commande 4a, on a incliné le support d'outils 5 pour l'orienter selon une direction d'inclinaison II.

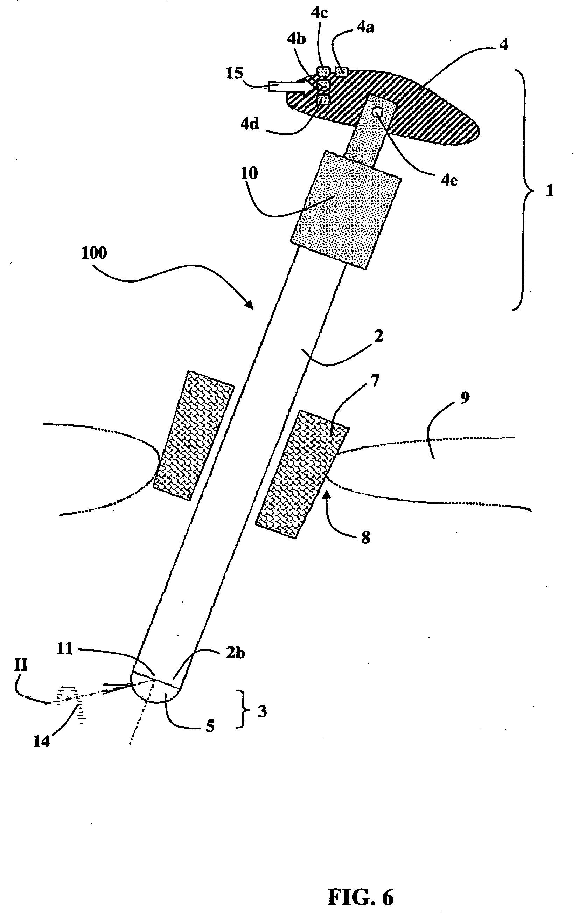

On considère maintenant la figure 6, qui illustre un second mouvement du support d'outils 5, à savoir un mouvement de rotation propre 14 autour de la direction d'inclinaison II. Ce mouvement de rotation propre 14 du support d'outils 5 autour de la direction d'inclinaison II est obtenu par une sollicitation 15 du second organe de commande 4b, qui commande un ou plusieurs actionneurs d'inclinaison logés dans le corps de commande 10.

La génération de mouvements de rotation propre 14 peut avantageusement être réalisée, selon l'invention, par la sollicitation 15 du second organe de commande 4b de la poignée 4, qui commande un actionneur de rotation propre 40b logé dans le corps de commande 10 (figure 17).

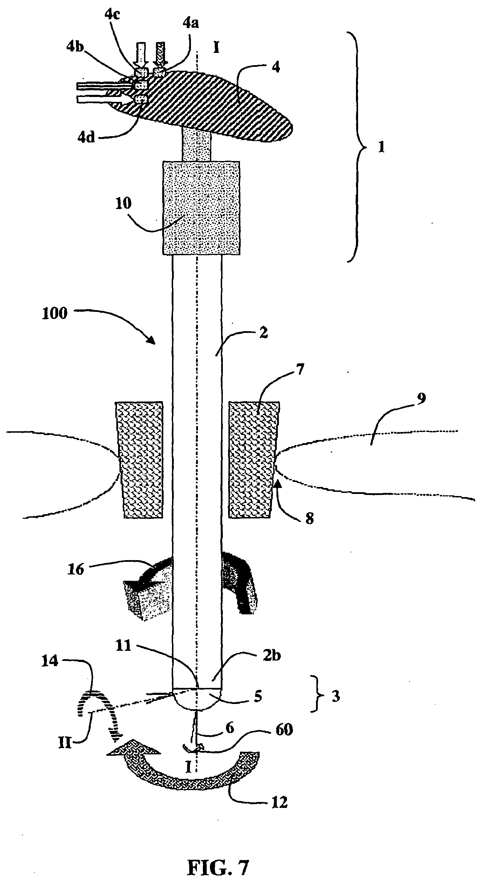

On considère maintenant la figure 7, qui illustre un autre mouvement du support d'outils 5, à savoir un mouvement par lequel on oriente la direction d'inclinaison II autour de l'axe longitudinal I-I du bras de liaison 2. Ce mouvement d'orientation est illustré par la flèche 16.

Selon l'invention, on peut produire ce mouvement d'orientation 16 soit en provoquant la rotation relative propre du bras de liaison 2 autour de son axe longitudinal I-I par rapport à la poignée 4, soit par une manipulation de la poignée 4 pour une rotation de l'ensemble du manipulateur autour de l'axe longitudinal I-I du bras de liaison 2.

Dans le premier cas, la rotation du bras de liaison 2 est obtenue par la sollicitation du troisième organe de commande 4c de la poignée 4, qui commande un actionneur de rotation de bras 40c logé dans le corps de commande 10 (figure 17).

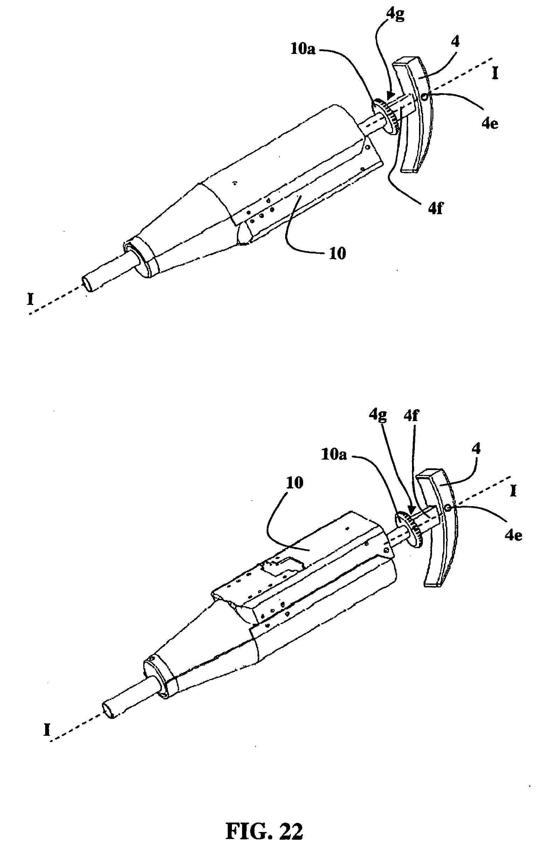

En variante de ce premier cas, la rotation du bras de liaison 2 peut être obtenue, comme illustré sur les figures 22 à 24, en prévoyant une articulation de rotation axiale relative de la poignée 4 sur le reste du manipulateur, avec des moyens d'indexation de la position relative de la poignée 4. En pratique, on peut prévoir que la poignée 4 est montée sur un support de poignée 4f lui-même articulé en rotation axiale relative sur le corps de commande 10. De préférence le corps de commande comprend une partie de préhension 10a que l'opérateur peut solliciter pour produire la rotation relative de la poignée 4 et du corps de commande 10.

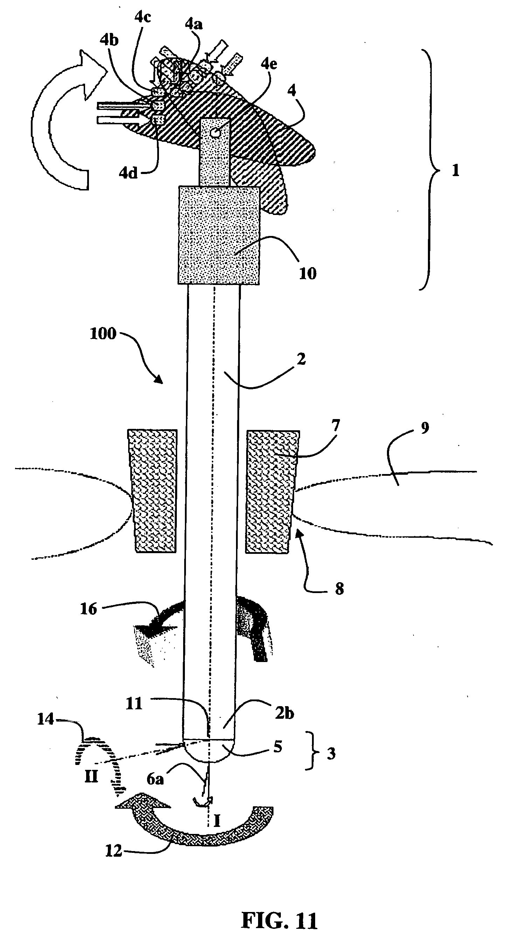

Dans le second cas, pour permettre une rotation axiale aisée de l'ensemble du manipulateur 100 par manipulation de la seule poignée 4, la poignée 4 est elle-même articulée autour d'un axe d'articulation de poignée 4e, comme illustré sur la figure 11.

Ainsi, sur la figure 7, on a illustré un premier mode de réalisation du manipulateur 100 selon lequel la poignée 4 comporte un premier organe de commande 4a dont la sollicitation provoque le mouvement d'inclinaison motorisé 12 du support d'outils 5, un second organe de commande 4b dont la sollicitation provoque le mouvement de rotation propre 14 du support d'outils 5 autour de sa direction d'inclinaison II, et un troisième organe de commande 4c pour produire le mouvement de rotation propre 16 du bras de liaison 2 autour de son axe longitudinal I-I. De la sorte, on peut orienter l'outil 6 dans toutes les directions d'un cône coiffant l'extrémité distale 2b du bras de liaison 2.

En outre, dans le mode de réalisation de la figure 7, l'outil 6 étant une pince, la poignée 4 comporte un organe de commande de pince 4d, assurant la commande du mouvement de serrage de pince illustré par la flèche 60. En pratique, le serrage 60 de la pince peut être réalisé par la sollicitation de l'organe de commande de pince 4d qui commande la rotation d'un actionneur de pince 40d logé dans l'unité de commande 10 (figure 17).

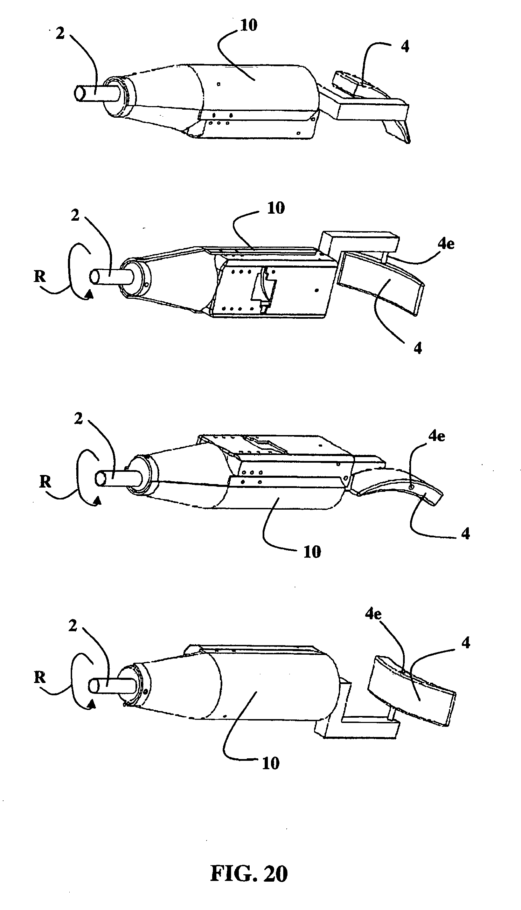

Selon la variante illustrée sur la figure 11, le bras de liaison 2 est solidaire du corps de commande 10, tandis que la poignée 4 est articulée autour d'un axe d'articulation 4e. Dans ce cas, le mouvement de rotation propre 16 est obtenu par rotation R (figure 20) de l'ensemble unité de commande 1 - bras de liaison 2, la rotation R étant produite par la main de l'opérateur qui tient la poignée 4. Une telle rotation R est illustrée par la suite des vues de la figure 20. L'articulation de la poignée 4 permet à la main de conserver une prise correcte de la poignée 4 sans mouvement exagéré de l'articulation du poignet, pendant une rotation suffisante du manipulateur autour de l'axe longitudinal I-I. Les autres mouvements du support d'outils 5 sont obtenus de façon motorisée, comme dans le mode de réalisation de la figure 7.

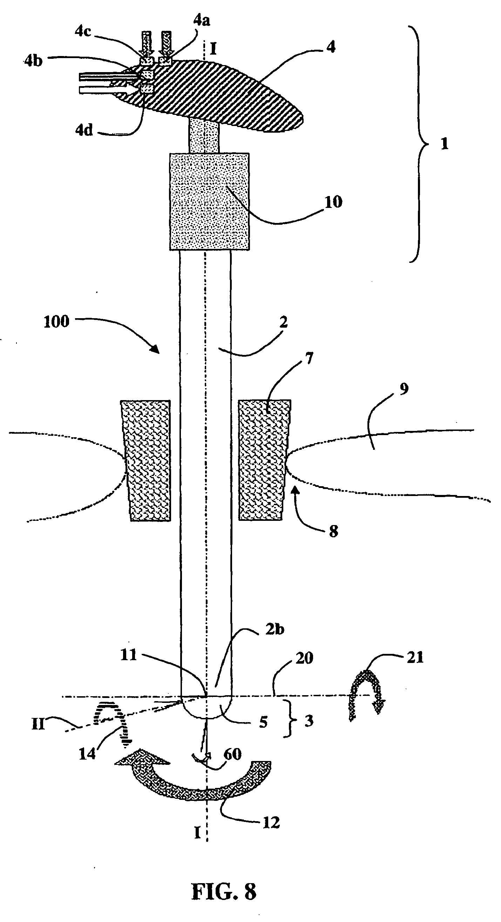

Dans le mode de réalisation de la figure 8, le mouvement d'orientation de la direction d'inclinaison II autour de l'axe longitudinal I-I du bras de liaison 2 est obtenu par sollicitation du troisième organe de commande 4c de la poignée 4, qui pilote un second actionneur d'inclinaison situé dans le corps de commande 10, lequel actionneur entraîne en rotation le support d'outils 5 selon un axe transversal concourant et perpendiculaire à l'axe transversal d'inclinaison 11 et à l'axe longitudinal I-I. Ce second axe transversal d'articulation est repéré par la référence numérique 20 sur la figure 8, et la rotation est illustrée par la flèche 21.

Ainsi, par combinaison des deux rotations d'inclinaison autour des axes 11 et 20, on peut orienter l'outil 6 dans toutes les orientations dans un cône coiffant l'extrémité distale 2b du bras de liaison 2.

Les autres mouvements du support d'outils 5 sont identiques à ceux des figures 7 et 11, et sont motorisés de la même façon.

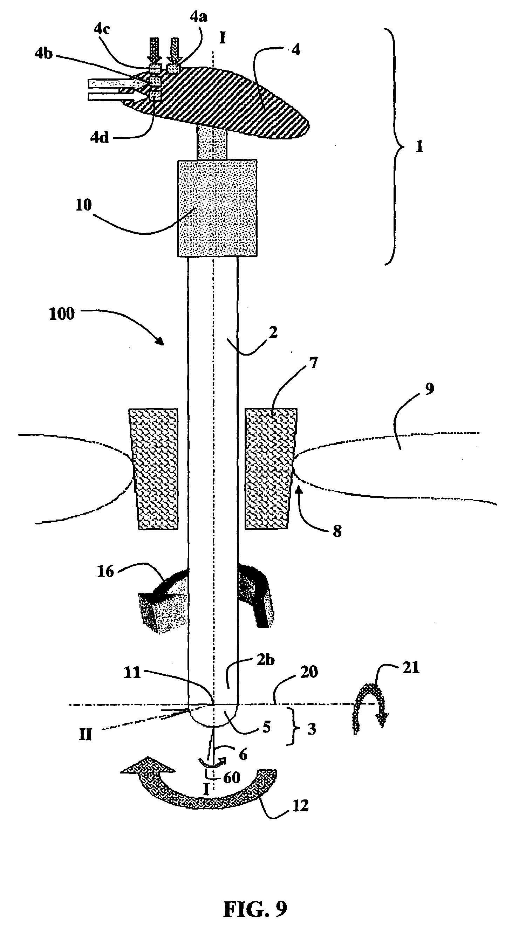

Dans le mode de réalisation illustré sur la figure 9, on retrouve le premier mouvement d'inclinaison 12 autour de l'axe transversal d'inclinaison 11, le mouvement de serrage de pince 60 pour l'outil 6, le second mouvement d'inclinaison par rotation 21 autour d'un second axe transversal 20 concourant, et le mouvement de rotation propre 16 du bras de liaison 2 autour de son axe longitudinal I-I. Dans ce cas, il n'est pas indispensable de prévoir d'autres moyens pour assurer la rotation propre de l'outil 6 autour de sa direction d'inclinaison II, car ce mouvement peut être réalisé par combinaison synchrone des rotations autour des axes 11, 20 et I-I.

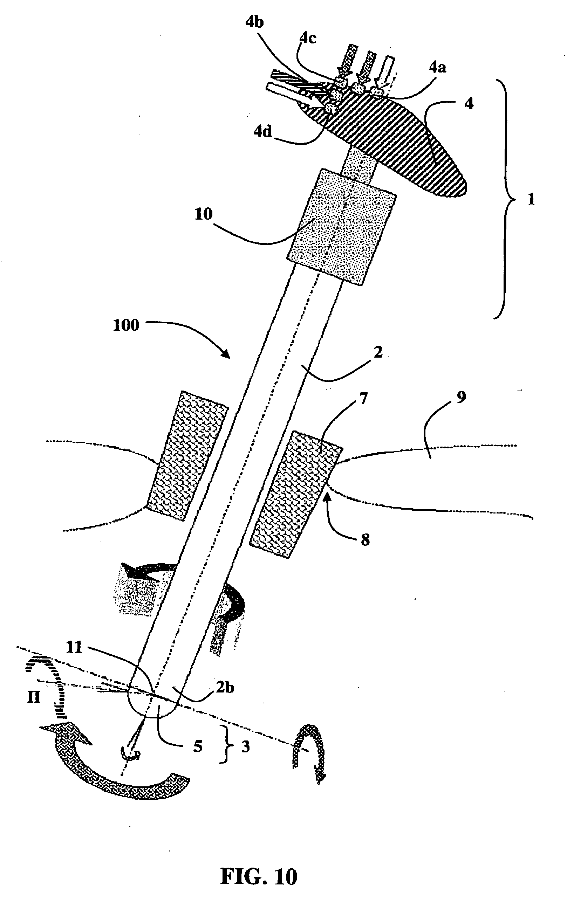

Toutefois, un mouvement de rotation propre de l'outil 6 autour de son axe d'inclinaison II peut avantageusement être ajouté, comme illustré sur la figure 10.

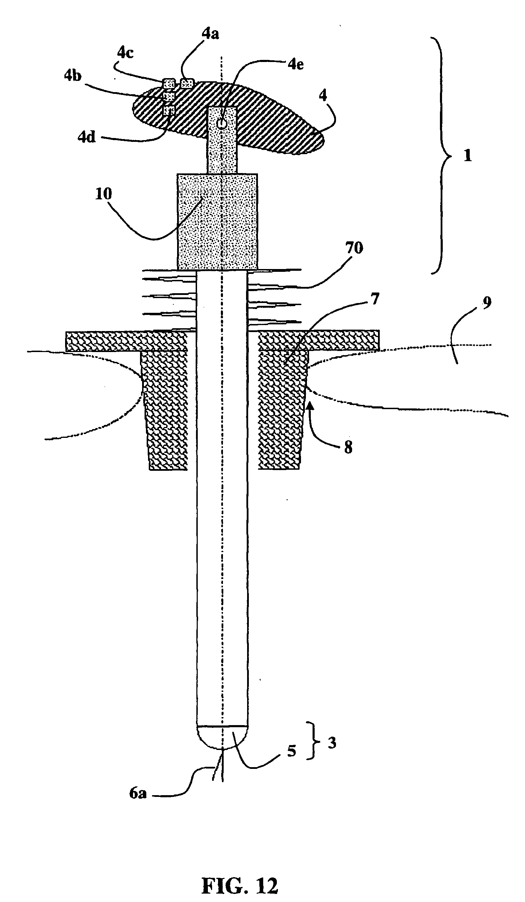

La figure 12 illustre une variante dans laquelle un système élastique 70 de compression est interposé entre le trocart 7 et le corps de commande 10, pour soulager l'opérateur d'une partie du poids du manipulateur.

Dans tous les modes de réalisation décrits ci-dessus, les organes de commandes 4a-4d peuvent être de type bouton-poussoir, bouton tactile, ou tout autre type de dispositif actionnable par un doigt selon une faible course et une faible force d'actionnement. De la sorte, on réduit encore le risque d'interférence entre l'actionnement des organes de commandes et le maintien en position de l'ensemble du manipulateur par la main qui porte la poignée 4.

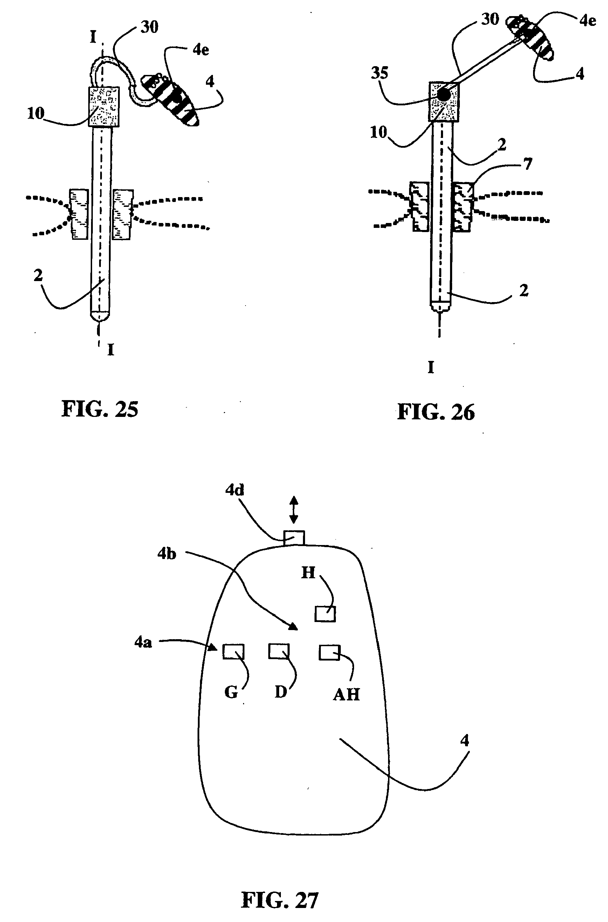

Selon un mode de réalisation avantageux, illustré par la figure 27, le second organe de commande 4b comprend un premier organe d'entrée H dont l'actionnement provoque la rotation propre du support d'outils 5 dans un premier sens horaire de rotation, et un second organe d'entrée AH dont l'actionnement provoque la rotation propre du support d'outils 5 dans un second sens anti-horaire de rotation. Par exemple, les organes d'entrée H et AH peuvent être deux boutons-poussoirs.

Ces boutons-poussoirs H et AH peuvent piloter chacun la rotation du support d'outils 5 selon une vitesse de rotation sensiblement constante.

En alternative, il peut s'agir de boutons-poussoirs de type progressifs, pilotant chacun la rotation du support d'outils 5 selon une vitesse de rotation variable entre une vitesse rapide et une vitesse lente.

Selon une autre possibilité plus avantageuse, permettant notamment de raccourcir les étapes préparatoires de conformation du manipulateur avant un geste opératoire, il peut s'agir de boutons-poussoirs de type « tout ou rien » à mode pas à pas se mettant en mode continu à vitesse plus rapide en cas de maintien de sollicitation.

En ce qui concerne le premier organe de commande 4a, on peut avantageusement le réaliser sous forme d'un troisième organe d'entrée D dont la sollicitation provoque une augmentation positive de l'angle d'inclinaison, et un quatrième organe d'entrée G dont la sollicitation provoque une augmentation négative de l'angle d'inclinaison. Par exemple, il peut s'agir de deux boutons-poussoirs G et D qui pilotent un moteur chacun dans un sens de rotation respectif, et le moteur s'arrête lorsqu'on relâche les boutons G et D.

Avantageusement, on peut en outre prévoir une possibilité de sollicitation de remise à zéro rapide, qui ramène le support d'outils 5 dans l'axe du bras de liaison 2, par exemple si l'on appuie sur les deux boutons-poussoirs G et D simultanément.

Quant à l'organe de commande de pince 4d, on peut le réaliser par exemple sous forme d'un bouton-poussoir à cliquet, qui commande le serrage de pince à la suite d'une première sollicitation d'appui, et qui commande l'ouverture de pince à la suite d'une seconde sollicitation d'appui, en pilotant pour cela l'actionneur de pince 40d dans l'un et l'autre des sens de rotation.

Avantageusement, on peut piloter l'actionneur de pince avec un organe de commande de pince 4d ayant une première position de pince ouverte, une seconde position de pince fermée à faible serrage, et une troisième position de pince fermée à fort serrage. Cela permet à l'opérateur, par exemple, de disposer une aiguille dans la pince, de piloter le faible serrage, de parfaire le positionnement de l'aiguille par glissement dans la pince, puis de bloquer l'aiguille en pilotant le fort serrage.

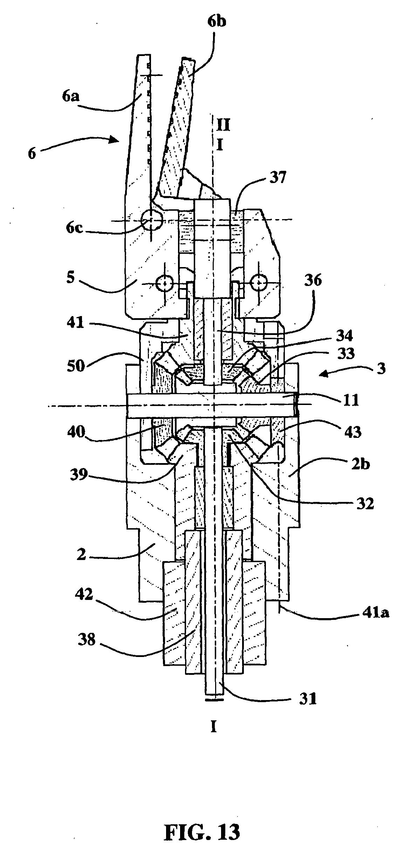

On considère maintenant les figures 13 et 15, qui illustrent un mode de réalisation de l'unité de travail 3, dans le cas d'un outil 6 en forme de pince ayant un mors fixe 6a et un mors mobile 6b autour d'un axe de rotation transversal 6c.

Pour le mouvement de serrage ou desserrage de la pince 6, on prévoit un arbre d'entrée 31 axial, couplé à l'actionneur de pince, orienté le long de l'axe longitudinal I-I du bras de liaison 2, portant un pignon conique d'extrémité 32 qui entraîne un pignon conique latéral 33 rotatif autour d'un axe transversal 11 et entraînant le pignon conique 34 lui-même monté en bout d'un arbre de sortie 36 fileté et calé axialement. Un écrou 37 est monté sur la partie fileté de l'arbre de sortie 36, et se déplace axialement lors de la rotation de l'arbre de sortie 36 pour entraîner en pivotement le mors mobile 6b de l'outil 6 autour de l'axe de rotation transversal 6c.

La rotation propre de l'outil 6 autour de l'axe d'inclinaison II est assurée par un tube d'entrée 38 orienté le long de l'axe longitudinal 1-1, entraîné lui-même en rotation par l'actionneur de rotation propre, et solidaire d'un pignon conique d'extrémité 39 qui lui-même entraîne en rotation un pignon conique latéral 40 rotatif autour d'un axe transversal 11 et qui entraîne un pignon conique axial 41 tubulaire solidaire du support d'outils 5 (solidaire du mors fixe 6a dans le cas présent). Autrement dit, l'outil 6 est monté avec son mors fixe 6a en position fixe sur le pignon 41, qui lui-même est monté rotatif axialement dans l'arceau 50. Un coussinet 42 guide le tube d'entrée 38 dans sa rotation autour de l'axe I-I dans le bras de liaison 2.

L'arceau 50 est monté pivotant autour de l'axe transversal 11 sur l'extrémité distale 2b du bras de liaison 2. Dans sa rotation, l'arceau 50 est solidaire d'une poulie 43 sollicitée en rotation par un câble 41 a, mieux visible sur la figure 15.

Comme on le distingue sur les figures 15 et 17, l'actionneur d'inclinaison 40a, de type moteur, produit par sa rotation l'entraînement du câble 41a et de la poulie 43, pour l'inclinaison de l'arceau 50 et du support d'outils 5 autour de l'axe transversal 11 jusqu'à une direction d'inclinaison II.

L'actionneur de rotation propre 40b assure la rotation propre du support d'outils 5 autour de la direction d'inclinaison II par entraînement du tube d'entrée 38. L'actionneur de pince 40d assure le serrage de la pince 6, par rotation de l'arbre d'entrée 31.

Sur la figure 17, on a en outre représenté un actionneur de rotation de bras 40c apte à provoquer la rotation de l'ensemble du manipulateur autour de la poignée 4.

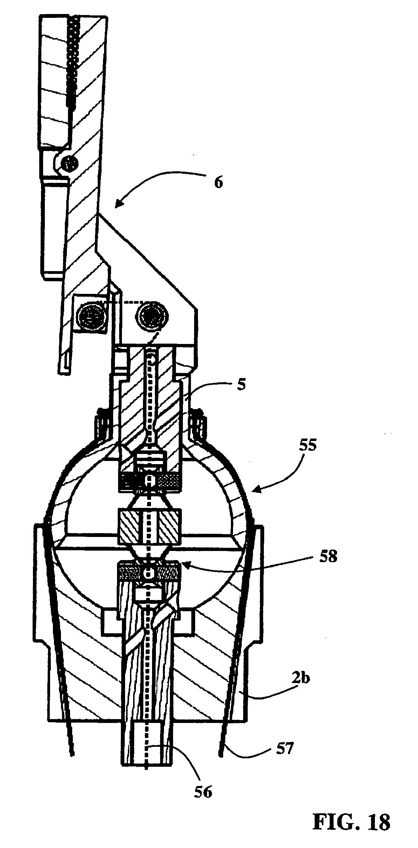

Dans la réalisation illustrée sur la figure 18, le support d'outils 5 est monté rotatif selon une articulation sphérique 55 sur l'extrémité distale 2b du bras de liaison 2. L'entraînement en orientation du support d'outils 5 est assuré par des câbles périphériques 57, pour un pivotement dans toutes les orientations possibles de la sphère.

La transmission du mouvement de serrage de la pince 6 s'effectue par un câble central 56, tandis que le mouvement de rotation propre s'effectue par une transmission par cardan 58. On pourra pour cela considérer la description de la demande de brevet français pendante N° 2 927 011, qui est insérée ici à titre de référence.

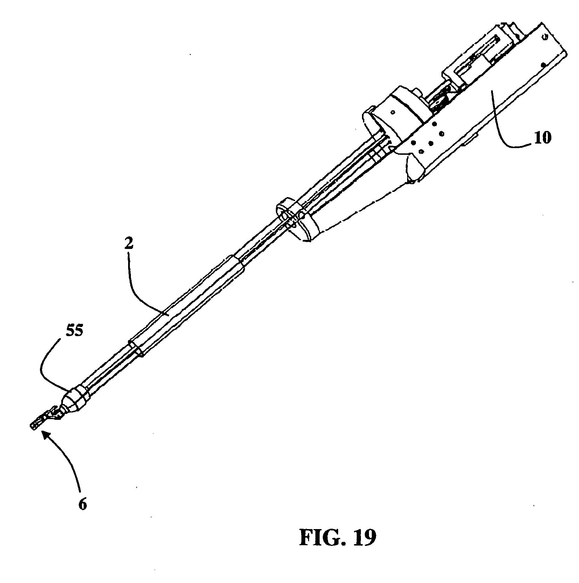

Sur la figure 19, on distingue l'ensemble du manipulateur selon ce mode de réalisation, avec des câbles pour la transmission des mouvements.

La figure 20 illustre le mouvement de rotation propre du manipulateur lui-même, dans le cas d'un mode de réalisation où la rotation propre du bras de liaison 2 n'est pas motorisée. Grâce à l'articulation de la poignée 4, la main peut suivre les mouvements sans que les doigts se détachent de la poignée 4, en assurant une rotation suffisante du manipulateur autour de son axe longitudinal.

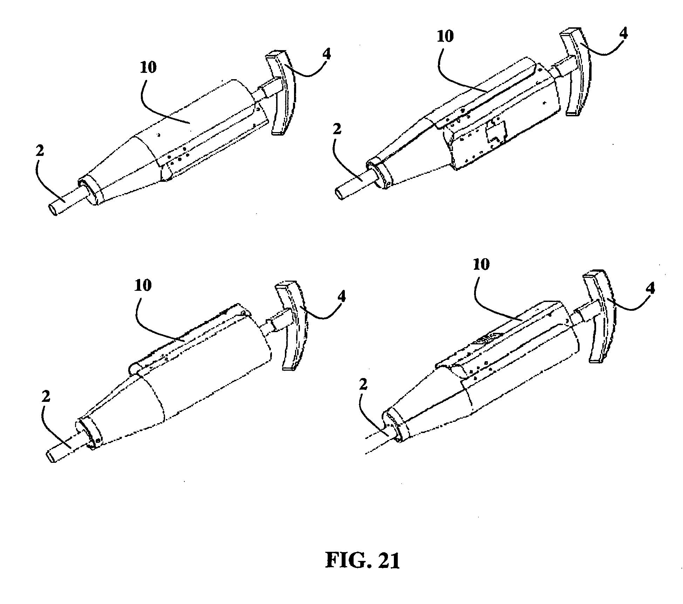

Sur les figures 21 et 17, on a illustré le mode de réalisation où le corps de commande 10 et le bras de liaison 2, solidaires l'un de l'autre, sont motorisés en rotation par rapport à la poignée 4.

Sur la figure 22, la poignée 4 est entraînée manuellement en rotation axiale autour de l'axe I-I grâce à une articulation axiale et une partie de préhension 10a sur le corps de commande 10. En pratique, la poignée 4 est articulée selon l'articulation transversale 4e sur un support de poignée 4f lui-même monté à rotation axiale libre par une articulation axiale 4g sur le corps de commande 10.

L'articulation axiale 4g et la partie de préhension 10a forment un organe d'entraînement manuel en rotation de la poignée 4 par rapport au reste du manipulateur autour de l'axe longitudinal I-I du bras de liaison 2. La sollicitation de la partie de préhension 10a, par un doigt de la main tenant la poignée 4, ou par une autre main, est utile pour réaliser les rotations de grande amplitude pour l'orientation de la direction d'inclinaison II autour de l'axe I-I. Les rotations de faible amplitude, au cours du geste opératoire, sont réalisées de façon ergonomique par sollicitation manuelle de la poignée 4 en rotation.

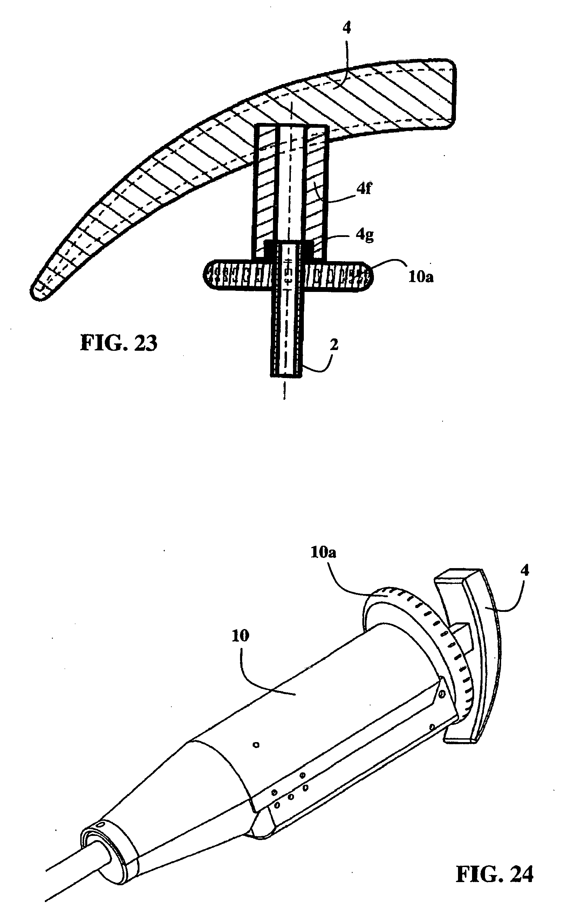

Dans la variante illustrée sur la figure 23, la poignée 4 est solidaire et fixe sur le support de poignée 4f. L'articulation 4g est un roulement dont la bague extérieure est solidaire du support de poignée 4 et dont la bague intérieure est solidaire du bras de liaison 2 et de la partie de préhension 10a en forme de molette calée sur le bras de liaison 2.

En alternative, dans la variante illustrée sur la figure 24, la partie de préhension 10a est directement solidaire du corps de commande 10.

Sur les figures 25 et 26, la poignée 4 est reliée au corps de commande 10 par un bras de positionnement 30, qui peut être utilisé indépendamment des autres caractéristiques décrites ici. Par ce bras de positionnement 30, la poignée 4 peut être orientée différemment, ou même déportée à l'écart de l'axe longitudinal I-I du manipulateur, pour une préhension plus ergonomique dans certaines conditions opératoires.

Sur la figure 25, le bras de positionnement 30 est une tige semi-rigide de 5 à 30 cm de long, déformable par flexion sous des forces de flexion ou de torsion plus importantes que les seules forces produites par le poids du manipulateur et la résistance des tissus traités par le manipulateur. La poignée 4 peut être fixe sur l'extrémité de la tige semi-rigide 30. En alternative, elle peut être articulée selon un axe d'articulation transversal 4e.

Sur la figure 26, le bras de positionnement 30 est une tige rigide, portant à son extrémité proximale la poignée 4 avec une éventuelle articulation transversale 4e, et articulé à son extrémité distale sur le corps de commande 10 par une articulation blocable 35.

Comme on l'a décrit dans certains modes de réalisation précédents, la poignée 4 peut avantageusement être montée libre en rotation autour d'un axe d'articulation 4e.

Par exemple, l'axe d'articulation 4e peut être un axe transversal par rapport à l'axe longitudinal I-I du bras de liaison 2.

On peut toutefois prévoir d'autres orientations de l'axe d'articulation 4e, par exemple une orientation longitudinale, ou une orientation oblique, chaque possibilité présentant des avantages respectifs.

Dans tous les modes de réalisation, chacun des actionneurs peut être par exemple de type moteur électrique, vérin hydraulique ou vérin pneumatique. On pourra préférer des actionneurs de type moteur électrique, notamment pour la facilité d'alimentation et la souplesse de commande.

La présente invention n'est pas limitée aux modes de réalisation qui ont été explicitement décrits, mais elle en inclut les diverses variantes et généralisations contenues dans le domaine des revendications ci-après.

## Revendications

1. Manipulateur comprenant,
- une unité de commande (1), ayant une poignée (4) apte à être tenue par une main,
- des organes de commande (4a-4d), montés sur la poignée (4), et aptes à être actionnés par au moins un doigt de la main tenant la poignée (4),
- un bras de liaison (2), s'étendant le long d'un axe longitudinal (I-I), ayant une extrémité proximale (2a) dans laquelle est montée l'unité de commande (1), et ayant une extrémité distale (2b),
- une unité de travail (3), montée sur l'extrémité distale (2b) du bras de liaison (2), et comportant un support d'outils (5) apte à supporter un outil (6),
- le support d'outils (5) étant monté inclinable dans l'unité de travail (3), par rapport au bras de liaison (2), par rotation autour d'un axe transversal d'inclinaison (11),
- les organes de commande (4a-4d) permettant de commander l'inclinaison (12) transversale du support d'outils (5) selon une direction d'inclinaison (II) par rotation autour de l'axe transversal d'inclinaison (11),
- les organes de commande (4a-4d) permettant de commander la rotation propre (14) du support d'outils (5) autour de la direction d'inclinaison (II),
et dans lequel :
- dans sa rotation d'inclinaison (12) transversale, le support d'outils (5) est entraîné par au moins un premier actionneur d'inclinaison (40a) piloté par un premier (4a) des organes de commande de la poignée (4),
- dans le mouvement de rotation propre (14) du support d'outils (5) autour de la direction d'inclinaison (II), le manipulateur est piloté par un second (4b) des organes de commande de la poignée (4), qui commande un ou plusieurs actionneurs (40b),
**caractérisé en ce que** les mouvements de rotation propre (14) et d'ouverture-fermeture (60) éventuelle d'outil (6) sont transmis au support d'outils (5) par des engrenages (39, 40, 41 ; 32, 33, 34) et tubes (38, 41) ou arbres (31, 36) logés dans l'unité de travail (3), tandis que les mouvements d'inclinaison autour de l'axe transversal d'inclinaison (11) sont transmis par des câbles (41 a).

2. Manipulateur selon la revendication 1, **caractérisé en ce que** le mouvement de rotation propre (14) du support d'outils (5) est assuré par un tube d'entrée (38) orienté le long de l'axe longitudinal (I-I), entraîné lui-même en rotation par un actionneur (40b) de rotation propre, et portant un pignon conique d'extrémité (39) qui lui-même entraîne en rotation un pignon conique latéral (40) rotatif autour d'un axe transversal (11) et qui entraîne un pignon conique axial (41) solidaire du support d'outils (5).

3. Manipulateur selon l'une des revendications 1 ou 2, dans lequel l'outil (6) est une pince, **caractérisé en ce que** :
- un actionneur de pince est couplé à un arbre d'entrée axial (31) orienté le long de l'axe longitudinal (I-I) du bras de liaison (2),
- l'arbre d'entrée axial (31) porte un pignon conique d'extrémité (32) qui entraîne un pignon conique latéral (33) rotatif autour d'un axe transversal (11) et entraînant un pignon conique (34) monté en bout d'un arbre de sortie (36) fileté et calé axialement sur lequel est monté un écrou (37) solidaire d'un mors mobile (6b) de l'outil (6).

4. Manipulateur selon la revendication 3, **caractérisé en ce que** la pince comprend un mors fixe (6a) et un mors mobile (6b).

5. Manipulateur selon l'une quelconque des revendications 1 à 4, **caractérisé en ce que** le support d'outils (5) comprend un arceau (50) monté pivotant autour d'un axe transversal (11) sur l'extrémité distale (2b) du bras de liaison (2) et solidaire d'une poulie (43) sollicitée en rotation par un câble (41a) lui-même sollicité par un actionneur d'inclinaison (40a).

6. Manipulateur selon l'une quelconque des revendications 1 à 5, **caractérisé en ce que** le second (4b) des organes de commande comprend un premier organe d'entrée (H) dont l'actionnement provoque la rotation propre du support d'outils (5) dans un premier sens de rotation, et un second organe d'entrée (AH) dont l'actionnement provoque la rotation propre du support d'outils (5) dans le second sens de rotation.

7. Manipulateur selon l'une quelconque des revendications 1 à 6, **caractérisé en ce que** le premier (4a) des organes de commande comprend un troisième organe d'entrée (D) dont la sollicitation provoque une augmentation positive de l'angle d'inclinaison, et un quatrième organe d'entrée (G) dont la sollicitation provoque une augmentation négative de l'angle d'inclinaison.

8. Manipulateur selon l'une des revendications 6 ou 7, **caractérisé en ce que** les organes d'entrée sont de type « tout ou rien », et pilotent chacun le mouvement du support d'outils (5) dans un sens respectif selon une vitesse de rotation sensiblement constante.

9. Manipulateur selon l'une des revendications 6 ou 7, **caractérisé en ce que** les organes d'entrée sont de type progressif, pilotant chacun le mouvement du support d'outils (5) selon une vitesse de rotation variable entre une vitesse rapide et une vitesse lente.

10. Manipulateur selon l'une des revendications 6 ou 7, **caractérisé en ce que** les organes d'entrée sont de type « tout ou rien » à mode pas à pas se mettant en mode continu à vitesse plus rapide par maintien de sollicitation.

11. Manipulateur selon la revendication 7, **caractérisé en ce que** le premier (4a) des organes de commande comprend en outre une possibilité de sollicitation de remise à zéro, qui ramène le support d'outils (5) dans l'axe du bras support.

12. Manipulateur selon l'une quelconque des revendications 1 à 11, **caractérisé en ce que**, dans le cas d'un outil (6) de type pince, le manipulateur comprend en outre, sur la poignée (4), un organe de commande de pince (4d) dont l'actionnement pilote au moins un actionneur de pince (40d).

13. Manipulateur selon la revendication 12, **caractérisé en ce que** l'organe de commande de pince (4d) comprend une première position de pince ouverte, au moins une seconde position de pince fermée à faible serrage, et au moins une troisième position de pince fermée à fort serrage.

14. Manipulateur selon l'une quelconque des revendications 1 à 13, **caractérisé en ce qu'**il comprend un actionneur de rotation de bras (40c) réalisant la rotation propre du bras de liaison (2) autour de son axe longitudinal (I-I) par rapport à la poignée (4), et réalisant ainsi un mouvement d'orientation (16) de la direction d'inclinaison (II) autour de l'axe longitudinal (I-I) du bras de liaison (2), ledit actionneur de rotation de bras étant piloté par le troisième organe de commande (4c) de la poignée (4).

15. Manipulateur selon l'une quelconque des revendications 1 à 13, **caractérisé en ce qu'**il comprend une articulation axiale (4g) entre le bras de liaison (2) et la poignée (4), et une partie de préhension (10a) telle qu'une molette solidaire du bras de liaison (2) ou du corps de commande (10), constituant un organe d'entraînement de la poignée (4) par rapport au reste du manipulateur pour réaliser un mouvement d'orientation (16) de la direction d'inclinaison (II) autour de l'axe longitudinal (I-I) du bras de liaison (2).

16. Manipulateur selon l'une quelconque des revendications 1 à 13 et 15, **caractérisé en ce que** la poignée (4) est articulée autour d'un axe d'articulation de poignée (4e).

17. Manipulateur selon l'une quelconque des revendications 1 à 14, **caractérisé en ce qu'**il comprend, entre la poignée (4) et le bras de liaison (2), un bras de positionnement (30) par lequel l'opérateur peut modifier et fixer la position angulaire et/ou spatiale relative à la poignée (4) par rapport au bras de liaison (2).

## Patentansprüche

1. Manipulator enthaltend,
- eine Steuereinheit (1), die einen Griff (4) aufweist, der ausgebildet ist, von einer Hand gehalten zu werden,
- Steuerorgane (4a-4d), die an dem Griff (4) angebracht sind und geeignet sind, von mindestens einem Finger der den Griff (4) haltenen Hand betätigt zu werden,
- einen Verbindungsarm (2), der sich längs einer Längsachse (I-I) erstreckt, ein proximales Ende (2a) aufweist, in dem die Steuereinheit (1) montiert ist, und der ein distales Ende (2b) aufweist,
- eine Arbeitseinheit (3), die an dem distalen Ende (2b) des Verbindungsarmes (2) angebracht ist und einen Werkzeughalter (5) aufweist, der in der Lage ist, ein Werkzeug (6) zu halten,
- wobei der Werkzeughalter (5) in der Arbeitseinheit (3) neigbar relativ zu dem Verbindungsarm (2) durch Drehung um eine transversale Neigungsachse (11) angebracht ist,
- wobei die Steuerorgane (4a-4d) ermöglichen, die transverse Neigung (12) des Werkzeughalters (5) in einer Neigungsrichtung (II) durch Drehung um die transverse Neigungsachse (11) zu steuern,
- wobei die Steuerorgane (4a-4d) es ermöglichen, eine genaue Drehung (14) des Werkzeughalters (5) um die Neigungsrichtung (II) zu steuern,
und wobei:
- der Werkzeughalter (5) bei seiner transversen geneigten Drehung (12) durch mindestens einen ersten Neigungsbetätigungsaktuator (40a) angetrieben ist, das durch ein erstes (4a) der Steuerorgane des Griffes (4) gesteuert ist,
- wobei der Manipulator bei der genauen Drehbewegung (14) des Werkzeughalters (5) um die Neigungsrichtung (II) durch ein zweites (4b) der Steuerorgane des Griffes (4) gesteuert ist, das einen oder mehrerer Aktuatoren (40b) steuert,
**dadurch gekennzeichnet, dass** die genauen Drehbewegungen (14) und mögliche Öffnungs-/Schließbewegungen (60) des Werkzeuges (6) zu dem Werkzeughalter (5) durch Getriebe (39, 40, 41; 32, 33, 34) und Rohre (38, 41) oder Wellen (31, 36) übertragen werden, die in der Arbeitseinheit (3) angeordnet sind, während die Neigungsbewegungen um die transversale Neigungsachse (11) durch Kabel (41a) übertragen werden.

2. Manipulator nach Anspruch 1, **dadurch gekennzeichnet, dass** die genaue Drehbewegung (14) des Werkzeughalters (5) durch ein Eingangsrohr (38) sichergestellt ist, das längs der Längsachse (I-I) ausgerichtet ist, das seinerseits durch einen eigenen Betätigungsaktuator (40b) für genaue Drehung angetrieben ist und einen konischen Endzapfen (39) trägt, der seinerseits einen konischen Seitenzapfen (40) zur Drehung um eine transversale Achse (11) antreibt und der einen konischen axialen Zapfen (41) antreibt, der mit dem Werkzeughalter (5) verbunden ist.

3. Manipulator nach einem der Ansprüche 1 oder 2, bei dem das Werkzeug (6) eine Zange ist, **dadurch gekennzeichnet, dass**:
- ein Betätigungsaktuator der Zange mit einem axialen Eingangsarm (31) gekoppelt ist, der längs der Längsachse (I-I) des Verbindungsarmes (2) ausgerichtet ist,
- wobei der axiale Eingangsarm (31) einen konischen Endzapfen (32) trägt, der einen konischen Seitenzapfen (33) drehbar um eine transversale Achse (11) antreibt und einen konischen Zapfen (34) antreibt, der am Ende eines Ausgangsarmes (36) angebracht ist, der ein Gewinde aufweist und axial festgesetzt ist und auf dem eine Mutter (37) angebracht ist, die mit einem beweglichen Schenkel (6b) des Werkzeuges (6) verbunden ist.

4. Manipulator nach Anspruch 3, **dadurch gekennzeichnet, dass** die Zange einen festen Schenkel (6a) und einen beweglichen Schenkel (6b) aufweist.

5. Manipulator nach irgendeinem der Ansprüche 1 bis 4, **dadurch gekennzeichnet, dass** der Werkzeughalter (5) einen Bügel (50) aufweist, der schwenkbar um eine transversale Achse (11) auf dem distalen Ende (2b) des Verbindungsarmes (2) angebracht ist und mit einer Rolle (43) verbunden ist, die durch ein Kabel (41a) drehbar angetrieben ist, das seinerseits durch einen Neigungs-Betätigungsaktuator (40a) angetrieben ist.

6. Manipulator nach irgendeinem der Ansprüche 1 bis 5, **dadurch gekennzeichnet, dass** das zweite (4b) der Betätigungsaktuatoren ein erstes Eingangsorgan (H) aufweist, dessen Betätigung eine genaue Drehung des Werkzeughalters (5) in einer ersten Drehrichtung bewirkt und ein zweites Eingangsorgan (AH), dessen Betätigung eine genaue Drehung des Werkzeughalters (5) in einer zweiten Drehrichtung bewirkt.

7. Manipulator nach irgendeinem der Ansprüche 1 bis 6, **dadurch gekennzeichnet, dass** das erste (4a) der Steuerorgane ein drittes Eingangsorgan (D) enthält, dessen Antrieb eine positive Vergrößerung des Neigungswinkels bewirkt und ein viertes Eingangsorgan (G), dessen Antrieb eine negative Vergrößerung des Neigungswinkels bewirkt.

8. Manipulator nach einem der Ansprüche 6 oder 7, **dadurch gekennzeichnet, dass** die Eingangsorgane des Typs "alles oder nichts" sind und jedes die Bewegung des Werkzeughalters (5) in entsprechender Richtung mit einer im wesentlichen konstanten Drehgeschwindigkeit steuert.

9. Manipulator nach einem der Ansprüche 6 oder 7, **dadurch gekennzeichnet, dass** die Eingangsorgane vom progressiven Typ sind, von denen jedes die Bewegung des Werkzeughalters (5) mit einer variablen Drehgeschwindigkeit zwischen einer schnellen und einer langsamen Geschwindigkeit steuert.

10. Manipulator nach einem der Ansprüche 6 oder 7, **dadurch gekennzeichnet, dass** die Eingangsorgane vom Typ "alles oder nichts" sind mit einer inkrementellen Betriebsart, die sich bei kontinuierlicher Betätigung zu einer Betriebsart mit höherer Geschwindigkeit ändert.

11. Manipulator nach Anspruch 7, **dadurch gekennzeichnet, dass** das erste (4a) der Steuerorgane zusätzlich eine Möglichkeit aufweist, die Betätigung auf Null zurückzusetzen, was den Werkzeughalter (5) zur Achse des Haltearmes zurückführt.

12. Manipulator nach irgendeinem der Ansprüche 1 bis 11, **dadurch gekennzeichnet, dass** im Falle, dass das Werkzeug (6) eine Zange ist, der Manipulator zusätzlich an dem Griff (4) ein Steuerorgan der Zange (4d) aufweist, dessen Betätigung mindestens einen Betätigungsaktuator der Zange (40d) steuert.

13. Manipulator nach Anspruch 12, **dadurch gekennzeichnet, dass** das Steuerorgan der Zange (4d) eine erste Öffnungsposition der Zange, mindestens eine zweite Schließposition der Zange mit schwacher Klemmung und mindestens eine dritte Position der geschlossene Zange mit starker Klemmung aufweist.

14. Manipulator nach irgendeinem der Ansprüche 1 bis 13, **dadurch gekennzeichnet, dass** er einen Betätigungsaktuator für die Drehung des Armes (40c) aufweist, die eine genaue Drehung des Verbindungsarmes (2) um seine Längsachse (I-I) in Bezug auf den Griff (4) realisiert und auch eine Ausrichtbewegung (16) der Neigungsrichtung (II) um die Längsachse (I-I) des Verbindungsarmes (2), wobei der Betätigungsaktuator für die Drehung des Armes durch ein drittes Steuerorgan (4c) des Griffes (4) gesteuert ist.

15. Manipulator nach irgendeinem der Ansprüche 1 bis 13, **dadurch gekennzeichnet, dass** er ein axiales Gelenk (4g) zwischen dem Verbindungsarm (2) und dem Griff (4) aufweist und ein Greifteil (10a), wie z.B. ein Rändelrad, das mit dem Verbindungsarm (2) oder dem Steuerkörper (10) verbunden ist, was ein Antriebsorgan des Griffes (4) in Bezug auf den Rest des Manipulators schafft, um eine Ausrichtbewegung (16) der Neigungsrichtung (II) um die Längsachse (I-I) des Verbindungsarmes (2) zu schaffen.

16. Manipulator nach irgendeinem der Ansprüche 1 bis 13 und 15, **dadurch gekennzeichnet, dass** der Griff (4) um eine Gelenkachse (4e) des Griffes schwenkbar ist.

17. Manipulator nach irgendeinem der Ansprüche 1 bis 14, **dadurch gekennzeichnet, dass** er zwischen dem Griff (4) und dem Verbindungsarm (2) einen Positionierungsarm (30) aufweist, mittels dessen der Operateur die Winkelposition und/oder relative Raumposition am Griff (4) in Bezug auf den Verbindungsarm (2) modifizieren und fixieren kann.

## Claims

1. Manipulator comprising,
- a control unit (1), having a handle (4) capable of being held by one hand,
- control members (4a-4d) mounted on the handle (4) and capable of being actuated by at least one finger of the hand holding the handle (4),
- a connecting arm (2) extending along a longitudinal axis (I-I), having a proximal end (2a) in which the control unit (1) is mounted, and having a distal end (2b),
- a working unit (3) mounted on the distal end (2b) of the connecting arm (2) and comprising a tool support (5) capable of supporting a tool (6),
- the tool support (5) being mounted in a manner in which it may be inclined in the working unit (3) relative to the connecting arm (2) by rotation about a transverse inclination axis (11),
- the control members (4a-4d) make it possible to control the transverse inclination (12) of the tool support (5) in a direction of inclination (II) by rotation about the transverse inclination axis (11),
- the control members (4a-4d) make it possible to control the specific rotation (14) of the tool support (5) about the direction of inclination (II),
and in which:
- in its transverse inclined rotation (12), the tool support (5) is driven by at least one first inclination actuator (40a) controlled by a first (4a) of the control members of the handle (4),
- in the specific rotational movement (14) of the tool support (5) about the direction of inclination (II), the manipulator is controlled by a second (4b) of the control members of the handle (4) which controls one or more actuators (40b),
**characterized in that** the specific rotational movements (14) and possible opening-closing (60) movements of the tool (6) are transmitted to the tool support (5) by gears (39, 40, 41; 32, 33, 34) and tubes (38, 41) or shafts (31, 36) housed in the working unit (3), whilst the inclination movements about the transverse axis of inclination (11) are transmitted by cables (41a).

2. Manipulator as claimed in claim 1, **characterized in that** the specific rotational movement (14) of the tool support (5) is provided by an input tube (38) oriented along the longitudinal axis (I-I), in turn driven in rotation by a specific rotation actuator (40b) and carrying a conical end pinion (39) which itself drives in rotation a lateral conical pinion (40) rotating about a transverse axis (11) and which drives an axial conical pinion (41) fixed to the tool support (5).

3. Manipulator as claimed in one of claims 1 or 2, in which the tool (6) is pliers, **characterized in that**:
- a pliers actuator is coupled to an axial input shaft (31) oriented along the longitudinal axis (I-I) of the connecting arm (2),
- the axial input shaft (31) carries a conical end pinion (32) which drives a lateral conical pinion (33) rotating about a transverse axis (11) and driving a conical pinion (34) mounted at the end of an axially threaded and wedged output shaft (36) on which is mounted a nut (37) fixed to a mobile jaw (6b) of the tool (6).

4. Manipulator as claimed in claim 3, **characterized in that** the pliers comprise a fixed jaw (6a) and a mobile jaw (6b).

5. Manipulator as claimed in any one of claims 1 to 4, **characterized in that** the tool support (5) comprises an arched portion (50) pivotably mounted about a transverse axis (11) on the distal end (2b) of the connecting arm (2) and fixed to a pulley (43) urged in rotation by a cable (41a) which is itself activated by an inclination actuator (40a).

6. Manipulator as claimed in any one of claims 1 to 5, **characterized in that** the second (4b) of the control members comprises a first input member (H), of which the actuation causes the specific rotation of the tool support (5) in a first rotational direction and a second input member (AH), of which the actuation causes the specific rotation of the tool support (5) in the second rotational direction.

7. Manipulator as claimed in any one of claims 1 to 6, **characterized in that** the first (4a) of the control members comprises a third input member (D), of which the activation causes a positive increase in the inclination angle, and a fourth input member (G), of which the activation causes a negative increase in the inclination angle.

8. Manipulator as claimed in one of claims 6 or 7, **characterized in that** the input members are of the "all or nothing" type and each control the movement of the tool support (5) in one respective direction according to a substantially constant rotational speed.

9. Manipulator as claimed in one of claims 6 or 7, **characterized in that** the input members are of the progressive type, each controlling the movement of the tool support (5) according to a rotational speed which is variable between a rapid speed and a slow speed.

10. Manipulator as claimed in one of claims 6 or 7, **characterized in that** the input members are of the "all or nothing" type in stepped mode, changing to continuous mode at higher speeds by maintaining the activation.

11. Manipulator as claimed in claim 7, **characterized in that** the first (4a) of the control members also has the potential for activating resetting, which returns the tool support (5) into the axis of the support arm.

12. Manipulator as claimed in any one of claims 1 to 11, **characterized in that** in the case of a pliers-type tool (6), the manipulator also comprises on the handle (4) a pliers control member (4d), of which the actuation controls at least one pliers actuator (40d).

13. Manipulator as claimed in claim 12, **characterized in that** the pliers control member (4d) comprises a first open pliers position, at least one second pliers position closed by light clamping and at least one third pliers position closed by strong clamping.

14. Manipulator as claimed in any one of claims 1 to 13, **characterized in that** it comprises an actuator for rotating the arm (40c), carrying out the specific rotation of the connecting arm (2) about its longitudinal axis (I-I) relative to the handle (4), and thus carrying out an orientation movement (16) of the direction of inclination (II) about the longitudinal axis (I-I) of the connecting arm (2), said actuator for rotating the arm being controlled by the third control member (4c) of the handle (4).

15. Manipulator as claimed in any one of claims 1 to 13, **characterized in that** it comprises an axial joint (4g) between the connecting arm (2) and the handle (4), and a gripping part (10a) such as a thumbwheel attached to the connecting arm (2) or to the control body (10), constituting a drive member of the handle (4) relative to the remainder of the manipulator to carry out an orientation movement (16) of the inclination direction (II) about the longitudinal axis (I-I) of the connecting arm (2).

16. Manipulator as claimed in any one of claims 1 to 13 and 15, **characterized in that** the handle (4) is articulated around an articulation axis (4e) of the handle.

17. Manipulator as claimed in any one of claims 1 to 14, **characterized in that** it comprises, between the handle (4) and the connecting arm (2), a positioning arm (30) by which the operator is able to modify and fix the angular and/or spatial position relative to the handle (4) with regard to the connecting arm (2).
